# EUROPÄISCHE PATENTSCHRIFT

(11) **EP 1 561 506 B1**
(45) Veröffentlichungstag und Bekanntmachung des Hinweises auf die Patenterteilung: **23.05.2007**
(21) Anmeldenummer: 05002208.6
(22) Anmeldetag: 03.02.2005
(51) Int. Cl.: B01D 53/70, B01D 53/85

(54) **Verfahren und Anordnung zur Reinigung von Faulgas**
Process and apparatus for biogas purification
Procédé et dispositif pour la purification de biogaz

(30) Priorität: 04.02.2004 DE 102004005626
(43) Veröffentlichungstag der Anmeldung: 10.08.2005
(73) Patentinhaber: Doczyck, Wolfgang, Dipl.-Ing., 46047 Oberhausen (DE)
(72) Erfinder: Doczyck, Wolfgang, Dipl.-Ing., 46047 Oberhausen (DE)
(74) Vertreter: Gesthuysen, von Rohr & Eggert

(56) Entgegenhaltungen:
- DE-A1- 19 920 258
- DE-C1- 10 158 804
- US-A- 5 899 187
- JARR M: "REINIGUNG VON DEPONIEGASEN MITTELS AKTIVKOHLE MIT ANSCHLIESSENDER BIOLOGISCHER REGENERATION" ENTSORGUNGS PRAXIS, BERTERSMANN FACHZEITSCHRIFTEN GMBH, DE, Bd. 15, Nr. 4, April 1997 (1997-04), Seiten 46-51, XP000684855 ISSN: 0724-6870

## Beschreibung

Die vorliegende Erfindung betrifft ein Verfahren zur Reinigung von Faulgas gemäß dem Oberbegriff des Anspruchs 1 sowie eine Anordnung zur Reinigung und Verbrennung von Faulgas gemäß dem Oberbegriff des Anspruchs 10.

Faulgase im Sinne der vorliegenden Erfindung entstehen beispielsweise in Kläranlagen, Biogasanlagen und Deponien durch anaerobe Umsetzung organischer Abfallstoffe. Sie enthalten neben Methan, Stickstoff und Kohlendioxid als Hauptbestandteile meist auch nennenswerte Anteile an halogenierten und nicht halogenierten Kohlenwasserstoffverbindungen sowie verschiedene Siliciumverbindungen unterschiedlicher Kettenlängen, insbesondere höher- und niedermolekulare Siloxane. Faulgase aus Kläranlagen enthalten hierbei überwiegend höhermolekulare, unpolare Siliciumverbindungen, während Deponiegas auch niedermolekulare Abbauprodukte, unter anderem Dimethylsilandiol und Tremethylsilanol, aufweist, die eher apolaren Charakter besitzen. Faulgase sind außerdem üblicherweise mit Wasserdampf gesättigt.

Seit langem wird Faulgas zum Betrieb von Gasmotoren, insbesondere in Kraft-Wärme-Kopplungsanlagen, eingesetzt. Diese Nutzung von Faulgas bereitet jedoch mit dem in den letzen Jahren zunehmenden Anteil an Siloxanen im Faulgas Probleme, da diese Siliciumverbindungen starken Verschleiß, hohen Aufwand sowie Schäden an den Gasmotoren verursachen. Die Siloxane führen im Verbrennungsprozeß zur Bildung von mineralischen Siliciumverbindungen. Diese können als Stäube im Zylinderraum sowie im Schmieröl wie ein Schleifmittel wirken und sich im Verbrennungsraum als glasartige Schichten anlagern, die gelegentlich abplatzen und große Schäden im Motor verursachen können. Die Reinigung von Faulgas zur Entfernung von Siliciumverbindungen, insbesondere Siloxanen, spielt daher für die Nutzung von Faulgas eine wesentliche Rolle.

Aus der DE 101 58 804 C1, die den Ausgangspunkt der vorliegenden Erfindung bildet, ist es bekannt, höhermolekulare Siliciumverbindungen katalytisch in niedermolekulare Siliciumverbindungen umzuwandeln und das Faulgas einer Gaswäsche zu unterziehen. Anschließend wird das Faulgas in einem Wärmetauscher erwärmt, um die relative Feuchte zu vermindern, und dann einem ggf, mehrstufigen Aktivkohlefilter zugeführt.

In der Praxis hat sich gezeigt, daß die Menge an erforderlicher Aktivkohle zur Abscheidung bzw. Adsorption von Verunreinigungen aus dem Faulgas oftmals beträchtlich ist. Dementsprechend sind mit der Bereitstellung der Aktivkohle, dem Austausch der Aktivkohle und der Entsorgung der beladenen Aktivkohle verhältnismäßig hohe Kosten verbunden.

Der vorliegenden Erfindung liegt die Aufgabe zugrunde, ein Verfahren zur Reinigung von Faulgas und eine Anordnung zur Reinigung und Verbrennung von Faulgas anzugeben, wobei eine besonders wirtschaftliche Reinigung, insbesondere eine Minimierung der erforderlichen bzw. auszutauschenden Menge an Adsorbentien, wie Aktivkohle, auf einfache Weise ermöglicht wird.

Die obige Aufgabe wird durch ein Verfahren gemäß Anspruch 1 oder eine Anordnung gemäß Anspruch 10 gelöst. Vorteilhafte Weiterbildungen sind Gegenstand der Unteransprüche.

Eine Idee der vorliegenden Erfindung liegt darin, adsorbierte Verunreinigungen mittels Unterdruck vom Adsorbens eines Filters, insbesondere eines Vorfilters, zumindest teilweise zu desorbieren. Insbesondere ist dem durch Unterdruck desorbierbaren Vorfilter ein Hauptfilter nachgeordnet. So kann wiederum eine wesentlich kostengünstigere Reinigung von Faulgas erreicht werden, da die Menge an erforderlichem bzw. auszutauschendem Adsorbens verringert werden kann.

Durch Adsorption lassen sich prinzipiell Siloxane aus dem Faulgas entfernen. Jedoch wird in der Praxis nur ein Bruchteil der theoretisch erzielbaren Beladungskapazität der Adsorbentien erreicht. Ursache hierfür sind die weiteren konkurrierenden Gasbestandteile des Faulgases, wie Wasserdampf und Kohlenwasserstoffe, die die Adsorbierbarkeit der Siloxane in den Adsorbentien drastisch einschränken. Bei voller Beladung des Vorfilters haben die konkurrierenden Gasbestandteile die Siloxane praktisch verdrängt. Die Siloxane bzw. sonstigen siliciumhaltigen Verbindungen werden dann im nachgeordneten Hauptfilter abgeschieden.

Die konkurrierenden Gasbestandteile können nun - im Gegensatz zu Siloxanen - mit verhältnismäßig hoher Desorptionsrate durch Unterdruck wieder desorbiert werden. Dementsprechend führt die zumindest teilweise Desorption von Verunreinigungen des Vorfilters durch Unterdruck zu einer einfachen, schnellen und kostengünstigen Vorreinigung, wobei der nachgeordnete Hauptfilter zumindest weitgehend von den genannten konkurrierenden Gasbestandteilen verschont bleibt und dadurch zumindest im wesentlichen lediglich zur Adsorption von Siloxanen und sonstigen Siliciumverbindungen mit hoher Beladungskapazität benutzt werden kann. Entsprechend ist das Adsorbens bzw. die Aktivkohle des Hauptfilters seltener als beim Stand der Technik auszutauschen, wodurch sich die Kosten und Wartungsintervalle reduzieren.

Ein weiterer Aspekt der vorliegenden Erfindung liegt darin, daß insbesondere bedarfsweise nur ein Teilstrom des Faulgases durch Filter mit einem Adsorbens, wie Aktivkohle, geleitet und anschließend mit dem Reststrom des Faulgases wieder zu einem Mischstrom gemischt wird, um den Verunreinigungsgrad des Mischstroms in ausreichender Weise reduzieren zu können, insbesondere um Grenzwerte für Verunreinigungen, insbesondere von Siliciumverbindungen, wie Siloxanen, für einen nachgeordneten Gasmotor einhalten zu können. Dies ermöglicht je nach Verunreinigungsgrad des ungereinigten Faulgases bzw. der eingesetzten Reinigungsstufen eine deutliche Verringerung der Menge an zu adsorbierenden Verunreinigungen und damit der erforderlichen bzw. auszutauschenden Menge an Adsorbentien. So werden eine verlängerte Standzeit der Adsorbentien, insbesondere der Aktivkohle, ein weniger häufiger Wechsel der Adsorbentien und eine geringere Menge an auszutauschenden und zu entsorgenden Adsorbentien mit dementsprechend geringeren Kosten ermöglicht.

Vorzugsweise wird das Verhältnis von Teilstrom zu Reststrom in Abhängigkeit vom Grad der Verunreinigung, insbesondere mit Siloxanen bzw. sonstigen Siliciumverbindungen, eingestellt bzw. variiert.

Besonders bevorzugt verläuft fortlaufend eine Messung von relevanten Verunreinigungen, wie Siliciumverbindungen, insbesondere Siloxanen, im Mischstrom, um einen entsprechenden Grenzwert für Verunreinigungen einhalten und dadurch einen nachgeordneten Gasmotor störungsfrei betreiben zu können, wobei gleichzeitig eine Reinigung nur insoweit wie erforderlich erfolgen kann.

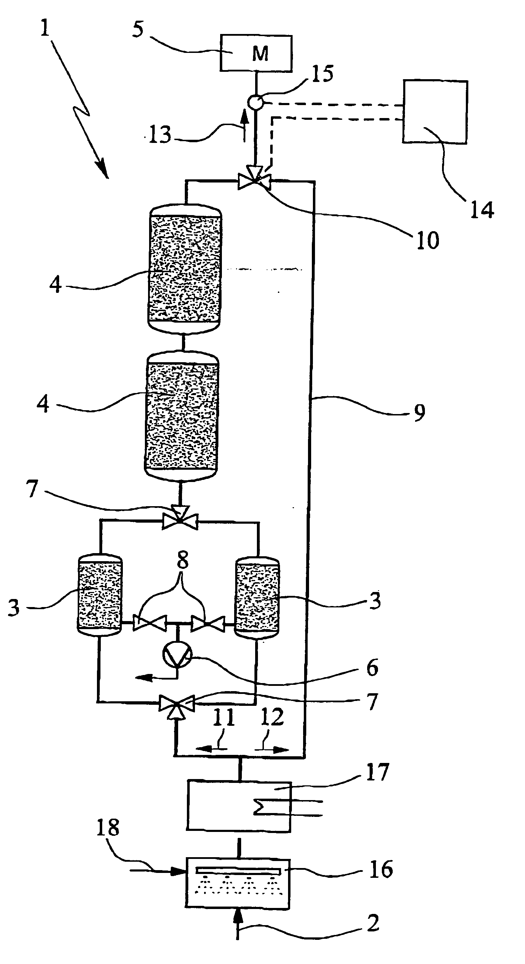

Weitere Vorteile, Merkmale, Eigenschaften und Aspekte der vorliegenden Erfindung ergeben sich aus der folgenden Beschreibung einer bevorzugten Ausführungsform anhand der Zeichnung. Die einzige Figur zeigt
eine schematische Darstellung einer vorschlagsgemäßen Anordnung zur Reinigung und Verbrennung von Faulgas.

Die vorschlagsgemäße Anordnung 1 zur Reinigung und Verbrennung von Faulgas 2 umfaßt Filter 3, 4, die beim Darstellungsbeispiel ein Adsorbens, ggf. auch unterschiedliche Adsorbentien, vorzugsweise Aktivkohle, zur Reinigung des Faulgases 2 enthalten.

Beim Darstellungsbeispiel sind zwei Filter 3 vorgesehen, die parallel geschaltet sind und Vorfilter bilden. Den Filtern 3 ist mindestens ein Filter 4 als Hauptfilter nachgeordnet. Beim Darstellungsbeispiel sind zwei Filter 4 vorgesehen, die in Reihe geschaltet sind und so verschiedene Filterstufen bilden.

Die vorschlagsgemäße Anordnung 1 weist weiter einen Gasmotor 5 auf, dem gereinigtes Faulgas 2 zur Verbrennung zuführbar ist. Bei dem Gasmotor 5 handelt es sich vorzugsweise um einen herkömmlichen bzw. angepaßten Innenverbrennungsmotor. Jedoch kann es sich hierbei in einem weiteren Sinne auch um eine Gasturbine, ein gasbetriebenes Heizkraftwerk, insbesondere ein Blockheizkraftwerk, eine sonstige Heizeinrichtung oder dgl. handeln.

Den beiden Vorfiltern 3 ist eine Unterdruckpumpe 6 zugeordnet. Durch entsprechende Umschaltventile 7 und Sperrventile 8 kann jeder Vorfilter 3 abwechselnd abgeschottet und mittels der Unterdruckpumpe 6 unter Unterdruck gesetzt werden, um adsorbierte Verunreinigungen, insbesondere Wasserdampf und Kohlenwasserstoffe, zumindest teilweise wieder von der Aktivkohle zu desorbieren, während zu reinigendes Faulgas 1 durch den jeweils anderen Vorfilter 3 geleitet wird. So wird eine kontinuierliche Vorreinigung ermöglicht.

Insbesondere bei entsprechender Abstimmung der Desorptionshäufigkeit, des Beladungsvolumens und der Abscheidungsleistung aufeinander ist es möglich, die Anordnung 1 derart zu betreiben, daß die Vorfilter 3 im wesentlichen nur mit zu Siloxanen konkurrierenden Gasbestandteilen beladen werden, die verhältnismäßig leicht bzw. effizient durch Unterdruck wieder desorbierbar sind, während die Hauptfilter 4 im wesentlichen nur mit Siloxanen und ggf. sonstigen Siliciumverbindungen beladen werden. Dies ermöglicht eine wesentlich bessere Ausnutzung der Beladungskapazität des Adsorbens bzw. der Aktivkohle mit Siliciumverbindungen in den Hauptfiltern 4 als beim Stand der Technik.

Vorzugsweise ist das Adsorbensvolumen der Hauptfilter 4 gegenüber den Vorfiltern 3 wesentlich größer, insbesondere mindestens um den Faktor 2, vorzugsweise 5 und ganz bevorzugt mindestens 10. Dies ermöglicht einen sehr effizienten Einsatz des Adsorbens bzw. der Aktivkohle, so daß bei verhältnismäßig geringen Kosten eine sehr gute Reinigungsleistung, insbesondere eine sehr effektive Abscheidung von Siloxanen und sonstigen Siliciumverbindungen, ermöglicht wird.

Gemäß einem weiteren Aspekt der vorliegenden Erfindung weist die vorschlagsgemäße Anordnung 1 eine Bypassleitung 9 und ein zugeordnetes Ventil 10 oder ein sonstiges Stellglied auf, um die die Filter 3, 4 umfassende Filterstufe bzw. die adsorbierende Reinigung bedarfsweise umgehen zu können. So wird ein Teilstrom 11 des Faulgases 2 durch die genannte Filterstufe bzw. die Filter 3, 4 geleitet und ein Reststrom 12 durch die Bypassleitung 9 um die Filterstufe bzw. die Filter 3, 4 herum geleitet. Die Ströme 11, 12 werden insbesondere mit dem Ventil 10 zu einem Mischstrom 13 zusammengeführt und dann dem Gasmotor 5 als gereinigtes Faulgas zugeleitet. Der Verunreinigungsgrad des Mischstroms 13 wird also in Abhängigkeit vom Verhältnis des Teilstroms 11 zum Reststrom 12 reduziert.

Das Ventil 10 ist beim Darstellungsbeispiel als Dreiwegeventil ausgebildet und insbesondere mittels einer zugeordneten Steuereinrichtung 14 derart einstellbar, daß das Verhältnis von Teilstrom 11 zu Reststrom 12 variierbar ist, insbesondere stufenlos oder in Stufen zwischen 0 und 1.

Bedarfsweise kann durch entsprechende Auslegung der Leitungen, Drosseln und/oder des Ventils 10 auch ein festes Verhältnis von Teilstrom 11 zu Reststrom 12 von beispielsweise 0,5 eingestellt bzw. festgelegt sein.

Zusätzlich oder alternativ zum angedeuteten Ventil 10 sind jedoch auch sonstige konstruktive Lösungen möglich. Insbesondere können ggf. einstellbare Druckminderer, Drosseln, Volumenstrommesser oder dgl. zur Steuerung bzw. Regelung des Verhältnisses von Teilstrom 11 zu Reststrom 12 eingesetzt werden.

Vorzugsweise wird der Verunreinigungsgrad, insbesondere der Anteil an Siloxanen und/oder sonstigen Siliciumverbindungen, im zu reinigenden Faulgas 2, im Teilstrom 11, im Reststrom 12 und/oder ganz besonders bevorzugt im Mischstrom 13 gemessen. Insbesondere weist die Anordnung 1 hierzu eine entsprechende Meßeinrichtung 15 auf Vorzugsweise erfolgt eine fortlaufende Messung bzw. Überwachung des Verunreinigungsgrads, insbesondere in der Zuleitung zum Gasmotor 5, wie dargestellt.

Gemäß einer besonders bevorzugten Variante ist die Meßeinrichtung 15 an die Steuereinrichtung 14 angeschlossen, so daß eine automatisierte Steuerung bzw. Regelung des Verhältnisses von Teilstrom 11 zu Reststrom 12 bzw. ein Öffnen bzw. Abschalten der Bypassleitung 9 erfolgen kann, beispielsweise in Abhängigkeit von Meßwerten und/oder bei Erreichen eines Grenzwerts.

Zusätzlich kann die Meßeinrichtung 15 zur Steuerung und/oder Anzeige eines Wechsels der Adsorbentien der Filter 3 bzw. 4 genutzt werden.

Beim Darstellungsbeispiel weist die vorschlagsgemäße Anordnung 1 zusätzlich eine Gaswäsche 16 auf. Die Gaswäsche 16 arbeitet vorzugsweise mit einem polaren Lösungsmittel, insbesondere Wasser. Ganz besonders bevorzugt ist Deponiesickerwasser 18 einsetzbar, das ohnehin bereits schadstoffbelastet ist und einer Nachbehandlung bedarf.

Weiter weist die Anordnung 1 eine der Gaswäsche 16 nachgeordnete Einrichtung zur Verringerung der relativen Feuchte des Faulgases 2, insbesondere einen Wärmetauscher 17, auf.

Das gewaschene und anschließend in seiner relativen Feuchte verminderte Faulgas 2 wird dann der genannten adsorbierenden Reinigung bzw. der Bypassleitung 9 zur direkten Weiterleitung an den Gasmotor 5 zugeführt.

Mit der vorschlagsgemäßen Anordnung 1 kann beispielsweise ausgehend von Faulgas 2 mit einem Gehalt an Siloxanen von 30 mg/m³ durch die Gaswäsche 16 zunächst eine Verringerung des Siloxangehalts auf etwa 15 mg/m³ erreicht werden. Wenn anschließend die Hälfte dieses Gasstroms als Teilstrom 11 durch die Filter 3, 4 geleitet wird, erfolgt aufgrund der quasi vollständigen Abscheidung der Siloxane im Teilstrom 11 eine weitere Halbierung des Siloxangehalts im Mischstrom 13 auf etwa 7,5 mg/m³. Damit kann der übliche Grenzwert von 10 mg/m³ an Siloxanen bzw. Siliciumverbindungen, die dem Gasmotor 5 zuführbar sind, ausreichend und mit einem vertretbaren gegenüber dem Stand der Technik wesentlich verringerten Aufwand an Adsorbens bzw. Aktivkohle unterschritten werden.

Die vorschlagsgemäße Anordnung 1 und das vorschlagsgemäße Verfahren gestatten also eine an den jeweiligen Einzelfall angepaßte optimale Reinigung im Hinblick auf die Einhaltung eines Grenzwerts für die Verunreinigung mit Siliciumverbindungen, insbesondere Siloxanen, für den Gasmotor 5, wobei eine optimale Ausnutzung der Beladungskapazität des Adsorbens, insbesondere der Aktivkohle, mit Siliciumverbindungen bzw. eine Minimierung der erforderlichen Menge an Adsorbens, insbesondere Aktivkohle, bezogen auf den Gesamtstrom an Faulgas 2 ermöglicht wird bzw. werden.

## Patentansprüche

1. Verfahren zur Reinigung von Siliciumverbindungen enthaltendem Faulgas (2) mittels eines Vorfilters (3) mit einem Adsorbens, vorzugsweise Aktivkohle, und eines nachgeschalteten Hauptfilters (4),
**dadurch gekennzeichnet,**
**daß** adsorbierte Siliciumverbindungen mittels Unterdruck vom Adsorbens zumindest teilweise desorbiert werden.

2. Verfahren nach Anspruch 1, **dadurch gekennzeichnet, daß** zwei Vorfilter (3) abwechselnd, vorzugsweise mittels der selben Unterdruckpumpe (6), unter Unterdruck gesetzt werden, während das Faulgas (2) durch den jeweils anderen Vorfilter (3) geleitet wird.

3. Verfahren nach einem der voranstehenden Ansprüche, **dadurch gekennzeichnet, daß** ein Teilstrom (11) des Faulgases (2) durch den bzw. die Filter (3, 4) geleitet und danach mit dem Reststrom (12) des nicht durch den bzw. die Filter (3, 4) geleiteten Faulgases (2) wieder zu einem Mischstrom (13) gemischt wird, um den Verunreinigungsgrad an Siliciumverbindungen im Mischstrom (13) in Abhängigkeit vom Verhältnis von Teilstrom (11) zu Reststrom (12) zu reduzieren.

4. Verfahren nach Anspruch 3, **dadurch gekennzeichnet, daß** der Grad der Verunreinigung mit Siliciumverbindungen im ungereinigten Faulgas (2), Teilstrom (11) und/oder Mischstrom (13), vorzugsweise fortlaufend gemessen wird.

5. Verfahren nach Anspruch 3 oder 4, **dadurch gekennzeichnet, daß** das Verhältnis von Teilstrom (11) zu Reststrom (12) fest eingestellt oder vorzugsweise in Abhängigkeit vom Grad der Verunreinigung des Mischstroms (13) mit Siliciumverbindungen variiert wird.

6. Verfahren nach einem der Ansprüche 3 bis 5, **dadurch gekennzeichnet, daß** das Verhältnis von Teilstrom (11) zu Reststrom (12) zwischen 0 und 1 stufenlos oder in Stufen variierbar ist.

7. Verfahren nach einem der voranstehenden Ansprüche, **dadurch gekennzeichnet, daß** das Faulgas (2) vor der adsorbierenden Reinigung einer Gaswäsche (16), insbesondere mit einem polaren Lösungsmittel, vorzugsweise Wasser, ganz bevorzugt Deponiesickerwasser (18), unterzogen wird.

8. Verfahren nach einem der voranstehenden Ansprüche, **dadurch gekennzeichnet, daß** die zumindest die relative Feuchte des Faulgases (2) vor der adsorbierenden Reinigung und insbesondere nach einer Gaswäsche (16) reduziert wird, vorzugsweise durch Erwärmen, insbesondere mittels eines Wärmetauschers (17).

9. Verfahren nach einem der voranstehenden Ansprüche, **dadurch gekennzeichnet, daß** der Mischstrom (13) einen verringerten Grad an Verunreinigungen mit Siliciumverbindungen aufweist und einem Gasmotor (5) zur Verbrennung zugeführt wird.

10. Anordnung (1) zur Reinigung von Siliciumverbindungen enthaltendem Faulgas (2) und anschließender Verbrennung, wobei die Anordnung (1) mindestens einen Filter (3, 4) mit einem Adsorbens, vorzugsweise Aktivkohle, und einen nachgeordneten Gasmotor (5) zur Verbrennung des gereinigten Faulgases (1) aufweist,
**dadurch gekennzeichnet,**
**daß** die Anordnung (1) eine Unterdruckpumpe (6) zur zumindest teilweisen Desorption von Siliciumverbindungen vom Adsorbens mindestens eines Filters (3) aufweist.

11. Anordnung nach Anspruch 10, **dadurch gekennzeichnet, daß** die Anordnung (1) eine Bypassleitung (9) und ein zugeordnetes Ventil (10) aufweist, so daß ein Teilstrom (11) des Faulgases (2) durch den bzw. die Filter (3, 4) leitbar und danach mit dem Reststrom (12) des nicht durch den bzw. die Filter (3, 4) geleiteten Faulgases (2) wieder zu einem Mischstrom (13) mischbar ist, um den Verunreinigungsgrad an Siliciumverbindungen im Mischstrom (13) in Abhängigkeit vom Verhältnis von Teilstrom (11) zu Reststrom (12) zu reduzieren, insbesondere wobei die Anordnung (1) eine Meßeinrichtung (15) aufweist, um den Grad der Verunreinigung mit Siliciumverbindungen im ungereinigten Faulgas (2), Teilstrom (11), Reststrom (12) und/oder Mischstrom (13) vorzugsweise fortlaufend zu messen.

12. Anordnung nach Anspruch 11, **dadurch gekennzeichnet, daß** das Verhältnis von Teilstrom (11) zu Reststrom (12) fest eingestellt oder vorzugsweise in Abhängigkeit vom Grad der Verunreinigung des Mischstroms (13) mit Siliciumverbindungen variierbar ist.

13. Anordnung nach Anspruch 11 oder 12, **dadurch gekennzeichnet, daß** das Verhältnis von Teilstrom (11) zu Reststrom (12) zwischen 0 und 1 stufenlos oder in Stufen variierbar ist.

14. Anordnung nach einem der Ansprüche 10 bis 13, **dadurch gekennzeichnet, daß** die Anordnung (1) zwei Vorfilter (3) aufweist, die abwechselnd mittels der Unterdruckpumpe (6) unter Unterdruck setzbar sind, während das Faulgas (2) durch den jeweils anderen Vorfilter (3) leitbar ist.

15. Anordnung nach Anspruch 14, **dadurch gekennzeichnet, daß** den Vorfiltern (3) mindestens ein Hauptfilter (4) nachgeordnet ist.

16. Anordnung nach Anspruch 15, **dadurch gekennzeichnet, daß** das Adsorbensvolumen des Hauptfilters (4) größer als das Adsorbensvolumen jedes Vorfilters (3) ist, insbesondere mindestens um den Faktor 2, vorzugsweise 5, insbesondere 10.

17. Anordnung nach einem der Ansprüche 10 bis 14, **dadurch gekennzeichnet, daß** die Anordnung (1) eine insbesondere mit Wasser, vorzugsweise Deponiesickerwasser (18), arbeitende Gaswäsche (16) aufweist.

18. Anordnung nach einem der Ansprüche 10 bis 15, **dadurch gekennzeichnet, daß** die Anordnung (1) eine Einrichtung, insbesondere einen Wärmetauscher (17), zur Reduzierung der relativen Feuchte des Faulgases (2) vor der adsorbierenden Reinigung und insbesondere nach einer Gaswäsche (16) aufweist.

## Claims

1. A method for purifying biogas (2) containing silicon compounds by means of a pre-filter (3) comprising an adsorbing substance, preferably activated charcoal, and a downstream main filter (4),
**characterised in**
**that** the adsorbed silicon compounds are at least partially desorbed from the adsorbing substance by means of vacuum.

2. The method according to claim 1, **characterised in that** two pre-filters (3) are alternately placed under vacuum, preferably by means of the same vacuum pump (6), whilst the biogas (2) is passed through the respectively other pre-filter (3).

3. The method according to any one of the preceding claims, **characterised in that** a partial stream (11) of the biogas (2) is passed through the filter or filters (3, 4) and is thereafter mixed again with the residual stream (12) of the biogas (2) which has not been passed through the filter or filters (3, 4) to form a mixed stream (13) in order to reduce the degree of contamination with silicon compounds in the mixed stream (13) depending on the ratio of the partial stream (11) to the residual stream (12).

4. The method according to claim 3, **characterised in that** the degree of contamination with silicon compounds in the unpurified biogas (2), partial stream (11) and/or mixed stream (13) is preferably measured continuously.

5. The method according to claim 3 or 4, **characterised in that** the ratio of partial stream (11) to residual stream (12) is fixedly set or is preferably varied depending on the degree of contamination of the mixed stream (13) with silicon compounds.

6. The method according to any one of claims 3 to 5, **characterised in that** the ratio of partial stream (11) to residual stream (12) can be varied between 0 and 1 continuously or in steps.

7. The method according to any one of the preceding claims, **characterised in that** before the adsorbing cleaning, the biogas (2) is subjected to gas scrubbing (16), in particular with a polar solvent, preferably water, quite preferably leachate (18).

8. The method according to any one of the preceding claims, **characterised in that** at least the relative humidity of the biogas (2) is reduced before the adsorbing cleaning and in particular after gas scrubbing (16), preferably by heating, in particular by means of a heat exchanger (17).

9. The method according to any one of the preceding claims, **characterised in that** the mixed stream (13) has a reduced degree of contamination with silicon compounds and is fed to a gas motor (5) for combustion.

10. An arrangement (1) for purifying biogas (2) containing silicon compounds and subsequent combustion, wherein the arrangement (1) comprises at least one filter (3, 4) comprising an adsorbing substance, preferably activated charcoal, and a downstream gas motor (5) for combustion of the purified biogas (1),
**characterised in**
**that** the arrangement (1) comprises a vacuum pump (6) for at least partial desorption of silicon compounds from the absorbing substance of at least one filter (3).

11. The arrangement according to claim 10, **characterised in that** the arrangement (1) comprises a bypass line (9) and an allocated valve (10) so that a partial stream (11) of the biogas (2) can be passed through the filter or filters (3, 4) and can then be mixed again with the residual stream (12) of the biogas (2) which has not been passed through the filter or filters (3, 4) to form a mixed stream (13) in order to reduce the degree of contamination with silicon compounds in the mixed stream (13) depending on the ratio of partial stream (11) to residual stream (12), in particular wherein the arrangement (1) comprises a measuring device (15) in order to measure, preferably continuously, the degree of contamination with silicon compounds in the unpurified biogas (2), partial stream (11), residual stream (12) and/or mixed stream (13).

12. The arrangement according to claim 11, **characterised in that** the ratio of partial stream (11) to residual stream (12) is fixedly set or preferably can be varied depending on the degree of contamination of the mixed stream (13) with silicon compounds.

13. The arrangement according to claim 11 or 12, **characterised in that** the ratio of partial stream (11) to residual stream (12) can be varied between 0 and 1 continuously or in steps.

14. The arrangement according to any one of claims 10 to 13, **characterised in that** the arrangement (1) comprises two pre-filters (3) which are alternately placed under vacuum by means of said vacuum pump (6), whilst the biogas (2) is passed through the respectively other pre-filter (3).

15. The arrangement according to claim 14, **characterised in that** at least one main filter (4) is located downstream of the pre-filters (3).

16. The arrangement according to claim 15, **characterised in that** the adsorbing volume of the main filter (4) is greater than the adsorbing volume of each pre-filter (3), in particular at least by a factor of 2, preferably 5, in particular 10.

17. The arrangement according to any one of claims 10 to 14, **characterised in that** the arrangement (1) comprises a gas scrubber (16) which operates in particular with water, preferably leachate (18).

18. The arrangement according to any one of claims 10 to 15, **characterised in that** the arrangement (1) comprises a device, in particular a heat exchanger (17), for reducing the relative humidity of the biogas (2) before the adsorbing cleaning and in particular after a gas scrubber (16).

## Revendications

1. Procédé pour la purification de biogaz (2) contenant des composés de silicium au moyen d'un premier filtre (3) avec un adsorbant, de préférence du charbon actif et un filtre principal (4) monté en aval,
**caractérisé en ce que**,
des composés de silicium adsorbés sont désorbés au moins partiellement de l'adsorbant, au moyen d'une dépression.

2. Procédé selon la revendication 1, **caractérisé en ce que** deux premiers filtres (3) sont placés alternativement sous dépression, de préférence au moyen de la même pompe à dépression (6), pendant que le biogaz (2) est conduit à travers chaque fois l'autre premier filtre (3).

3. Procédé selon l'une quelconque des revendications précédentes, **caractérisé en ce qu'**un flux partiel (11) du biogaz (2) est conduit à travers les filtres (3, 4), pour être remélangé ensuite avec le flux résiduaire (12) du biogaz (2) qui n'est pas conduit à travers les filtres (3, 4) en un flux mixte (13), pour réduire le taux de pollution en composés de silicium dans le flux mixte (13), en fonction du rapport du flux partiel (11) aux flux résiduaire (12).

4. Procédé selon la revendication 3, **caractérisé en ce que** le taux de pollution en composés de silicium est mesuré de préférence en continu dans le biogaz (2) non purifié, dans le flux partiel (11) et/ou dans le flux mixte (13).

5. Procédé selon la revendication 3 ou 4, **caractérisé en ce que** le rapport du flux partiel (11) au flux résiduaire (12) est fixement réglé ou qu'on le fait varier de préférence en fonction du taux de pollution du flux mixte (13) avec des composés de silicium.

6. Procédé selon l'une quelconque des revendications 3 à 5, **caractérisé en ce que** le rapport du flux partiel (11) au flux résiduaire (12) est variable directement ou par étapes entre 0 et 1.

7. Procédé selon l'une quelconque des revendications précédentes, **caractérisé en ce que**, avant la purification par adsorption, le biogaz (2) est soumis à un lavage du gaz (16), notamment avec un solvant polaire, de préférence de l'eau, de façon totalement préférée, de l'eau d'infiltration de décharge (18).

8. Procédé selon l'une quelconque des revendications précédentes, **caractérisé en ce qu'**on réduit au moins l'humidité relative du biogaz (2) avant la purification par adsorption et notamment après un lavage du gaz (16), de préférence par échauffement, notamment au moyen d'un échangeur thermique(17).

9. Procédé selon l'une quelconque des revendications précédentes, **caractérisé en ce que** le flux mixte (13) présente un taux réduit de pollution par composés de silicium et **en ce qu'**il est alimenté vers un moteur à gaz (5) pour la combustion.

10. Agencement (1) pour la purification de biogaz (2) contenant des composés de silicium et pour une combustion consécutive, l'agencement (1) comportant au moins un filtre (3, 4) avec un adsorbant, de préférence du charbon actif, et avec un moteur à gaz (5) monté en aval, pour la combustion du biogaz (1) purifié,
**caractérisé en ce que**,
l'agencement (1) comporte une pompe de dépression (6) pour la désorption au moins partielle de composés de silicium de l'adsorbant d'au moins un filtre (3).

11. Agencement selon la revendication 10, **caractérisé en ce que** l'agencement (1) comporte un conduit de dérivation (9) et une soupape (10) associée, pour qu'un flux partiel (11) du biogaz (2) puisse être conduit à travers le ou les filtre(s) (3, 4) et puisse être ensuite remélangé avec le flux résiduaire (12) du biogaz (2) qui n'est pas conduit à travers le ou les filtre(s) (3, 4) en un flux mixte (13), pour réduire le taux de pollution en composés de silicium dans le flux mixte (13) en fonction du rapport du flux partiel (11) au flux résiduaire (12), l'agencement (1) comportant notamment un dispositif de mesure (15) pour mesurer de préférence en continu le taux de la pollution en composés de silicium dans le biogaz (2) non purifié, dans le flux partiel (11), dans le flux résiduaire (12) et/ou dans le flux mixte (13).

12. Agencement selon la revendication 11, **caractérisé en ce que** le rapport du flux partiel (11) au flux résiduaire (12) est fixement réglé ou qu'il est variable de préférence en fonction du taux de pollution du flux mixte (13) avec des composés de silicium.

13. Agencement selon la revendication 11 ou 12, **caractérisé en ce que** le rapport du flux partiel (11) au flux résiduaire (12) est variable directement ou par étapes entre 0 et 1.

14. Agencement selon l'une quelconque des revendications 10 à 13, **caractérisé en ce que** l'agencement (1) comporte deux premiers filtres (3) qui peuvent être placés alternativement sous dépression, au moyen de la pompe à dépression (6), pendant que le biogaz (2) peut être conduit à travers chaque fois l'autre premier filtre (3).

15. Agencement selon la revendication 14, **caractérisé en ce que** au moins un filtre principal (4) est monté en aval des premiers filtres (3).

16. Agencement selon la revendication 15, **caractérisé en ce que** le volume d'adsorbant du filtre principal (4) est supérieur au volume d'adsorbant de chaque premier filtre (3), notamment au moins de la valeur du facteur 2, de préférence 5, notamment 10.

17. Agencement selon l'une quelconque des revendications 10 à 14, **caractérisé en ce que** l'agencement (1) comporte un lavage de gaz (16), fonctionnant notamment avec de l'eau, de préférence avec de l'eau d'infiltration de décharge (18).

18. Agencement selon l'une quelconque des revendications 10, à 15, **caractérisé en ce que** l'agencement (1) comporte un dispositif, notamment un échangeur thermique (17), pour réduire l'humidité relative du biogaz (2) avant la purification par adsorption et notamment après un lavage du gaz (16).
